(19) Europäisches Patentamt
European Patent Office
Office européen des brevets

(11) Veröffentlichungsnummer: **0 058 370**
**B1**

(12) # EUROPÄISCHE PATENTSCHRIFT

(45) Veröffentlichungstag der Patentschrift: **12.12.84**

(21) Anmeldenummer: **82100901.6**

(22) Anmeldetag: **08.02.82**

(51) Int. Cl.³: **C 07 C 143/36,** C 07 C 143/78, C 07 C 145/00, C 07 C 147/06, C 07 C 149/30, C 07 C 147/14, C 07 C 143/68, C 07 C 149/28, A 61 K 31/10, A 61 K 31/16

(54) **Tetrahydronaphthalin- und Indanverbindungen, ihre Herstellung und pharmazeutische Präparate.**

(30) Priorität: **13.02.81 CH 974/81**
**09.11.81 CH 7175/81**

(43) Veröffentlichungstag der Anmeldung: **25.08.82 Patentblatt 82/34**

(45) Bekanntmachung des Hinweises auf die Patenterteilung: **12.12.84 Patentblatt 84/50**

(84) Benannte Vertragsstaaten: **AT BE CH DE FR GB IT LI LU NL SE**

(56) Entgegenhaltungen: **Keine**

(73) Patentinhaber: **F. HOFFMANN-LA ROCHE & CO. Aktiengesellschaft, CH-4002 Basel (CH)**

(72) Erfinder: **Klaus, Michael, Dr., Am Hellenrain 6, CH-7858 Weil/Rhein (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft neue Tetrahydronaphthalin- und Indanverbindungen der Formel

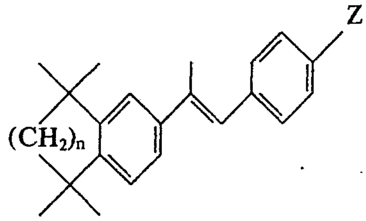

(I)

worin n 1 oder 2; Z Mercapto oder einen Rest $-S(O)_mR$, m 0, 1 oder 2; R nieder-Alkyl, nieder-Alkenyl, nieder-Alkoxy-nieder-alkyl, nieder-Alkanoyl-nieder-alkyl, Hydroxy-nieder-alkyl, Halogen-nieder-alkyl, nieder-Carbalkoxy-nieder-alkyl oder, falls m 1 oder 2 ist, auch nieder-Alkoxy, Hydroxy, mono- oder di-nieder-Alkylamino bedeutet,
und pharmazeutisch verträgliche Salze von Sulfon- und Sulfinsäuren der Formel I.

Die Erfindung betrifft weiterhin ein Verfahren zur Herstellung der Verbindungen der Formel I, Zwischenprodukte in diesem Verfahren und pharmazeutische Präparate auf der Basis von Verbindungen der Formel I.

Der hier verwendete Ausdruck »nieder« bezeichnet Gruppen mit vorzugsweise 1—4 C-Atomen. Alkylgruppen können geradkettig oder verzweigt sein. Bevorzugte nieder-Alkylgruppen sind Methyl, Äthyl und Isopropyl. Beispiele von nieder-Alkenylgruppen sind Vinyl, Allyl und Methallyl. Beispiele von nieder-Alkanoylresten sind Acetyl, Propionyl und Butyryl. Halogen umfaßt Fluor, Chlor, Brom und Jod, wovon Chlor bevorzugt ist. Beispiele von nieder-Carbalkoxy-nieder-alkyl sind Carbomethoxy- und Carboäthoxymethyl und -äthyl. Beispiele von nieder-Alkoxy sind Methoxy und Äthoxy, Beispiele von Alkylaminoresten sind Methylamino, Äthylamino, Isopropylamino, Dimethylamino und Diäthylamino. Als pharmazeutisch verträgliche Salze kommen z. B. Alkalimetall- und Erdalkalimetallsalze sowie Ammoniumsalze in Betracht.

Von den Verbindungen der Formel I sind solche in denen R nieder-Alkyl, nieder-Alkenyl, Hydroxy-nieder-alkyl, Halogen-nieder-alkyl, nieder-Carbalkoxy-nieder-alkyl oder, falls m 1 oder 2 ist, auch nieder-Alkoxy, Hydroxy, mono- oder di-nieder-Alkylamino bedeutet, bevorzugt.

Bevorzugte Verbindungen der Formel I sind ferner solche, in denen n=2 ist. Weiterhin sind Verbindungen der Formel I mit m=2 bevorzugt, sowie solche, in denen R nieder-Alkyl, Hydroxy, nieder-Alkoxy, nieder-Alkylamino, Hydroxy-nieder-alkyl oder nieder-Carbalkoxy-nieder-alkyl ist.

Von besonderem Interesse sind die Verbindungen
Äthyl-p-[2-(5,6,7,8-tetrahydro-5,5,8,8-tetramethyl-2-naphthyl)propenyl]benzolsulfonat;
Äthyl-p-[2-(5,6,7,8-tetrahydro-5,5,8,8-tetramethyl-2-naphthyl)-propenyl]-phenylsulfon;
Äthyl-p-[2-(5,6,7,8-tetrahydro-5,5,8,8-tetramethyl-2-naphthyl)-propenyl]-phenylsulfoxid;
Äthyl-p-[2-(5,6,7,8-tetrahydro-5,5,8,8-tetramethyl-2-naphthyl)-propenyl]-phenylsulfid;
Isopropyl-p-[2-(5,6,7,8-tetrahydro-5,5,8,8-tetramethyl-2-naphthyl)-propenyl]-phenylsulfon.

Die Verbindungen der Formel I können erfindungsgemäß dadurch hergestellt werden, daß man

a) eine Verbindung der Formel

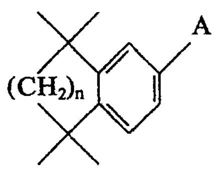

(II)

mit einer Verbindung der Formel

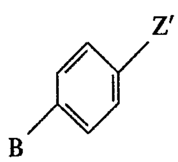

(III)

in denen n die oben angegebene Bedeutung hat, Z' $-SO_2^-M^+$, $-SO_3^-M^+$, nieder-Alkylthio, nieder-Alkylsulfinyl oder nieder-Alkylsulfonyl und entweder A eine Triarylphosphoniumäthylgruppe der Formel

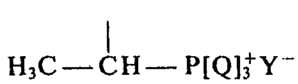

und B Formyl ist; oder A Acetyl und B eine Dialkoxyphosphonylmethylgruppe der Formel

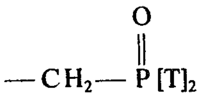

ist; wobei Q Aryl, T nieder-Alkoxy, $Y^-$ das Anion einer organischen oder anorganischen Säure und $M^+$ ein Kation bezeichnen,
umsetzt, oder

b) eine Verbindung der Formel

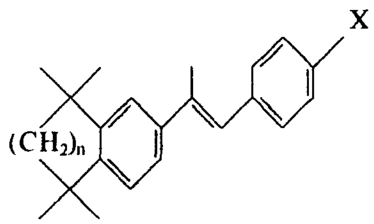

(IV)

in der n die obige Bedeutung hat und X Halogen ist,
in Gegenwart einer starken Base mit $SO_2$ umsetzt, oder

c) eine Verbindung der Formel

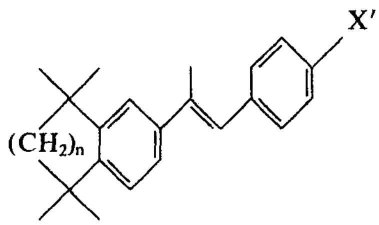

(V)

in der n die obige Bedeutung hat und X' di-nieder-Alkylcarbamoylthio ist,
mit einer Base behandelt oder mit einem komplexen Metallhydrid reduziert, und gewünschtenfalls in einer gemäß a, b oder c erhaltenen Verbindung der Formel I den Rest Z funktionell abgewandelt.

Die Umsetzung gemäß Verfahrensvariante a) kann in für die Wittig- und Horner-Reaktion an sich bekannter Weise bzw. unter den für diese Reaktionen an sich bekannten Bedingungen durchgeführt werden.

Bei der Umsetzung einer Verbindung der Formel II, in der A eine Triarylphosphoniumäthylgruppe darstellt, mit einer Verbindung der Formel III (Wittig-Reaktion) werden die Komponenten in Gegenwart eines säurebindenden Mittels, z. B. in Gegenwart einer starken Base, wie z. B. Butyllithium, Natriumhydrid oder dem Natriumsalz von Dimethylsulfoxyd, vornehmlich aber in Gegenwart eines gegebenenfalls durch niederes Alkyl substituierten Äthylenoxyds, wie 1,2-Butylenoxyd, gegebenenfalls in einem Lösungsmittel, z. B. in einem Äther, wie Diäthyläther oder Tetrahydrofuran, oder in einem aromatischen Kohlenwasserstoff, wie Benzol, in einem zwischen der Raumtemperatur und dem Siedepunkt des Reaktionsgemisches liegenden Temperaturbereich miteinander umgesetzt.

Die in den genannten Triarylphosphoniumgruppen mit Q bezeichneten Arylgruppen umfassen gemeinhin alle bekannten Arylreste, insbesondere aber einkernige Reste, wie Phenyl oder nieder-Alkyl, bzw. nieder-Alkoxy-substituiertes Phenyl, wie Toluol, Xylyl, Mesityl oder p-Methoxyphenyl.

Von den anorganischen Säureanionen Y ist das Chlor- und Brom-ion oder das Hydrosulfat-ion, von den organischen Säureanionen ist das Tosyloxy-ion bevorzugt.

Die in der Dialkoxyphosphonylmethylgruppe der Formel

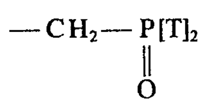

mit T bezeichneten Alkoxyreste sind vornehmlich niedere Alkoxyreste mit 1—6 Kohlenstoffatomen, wie Methoxy oder Äthoxy.

Bei der Umsetzung einer Verbindung der Formel II, in der A Acetyl ist, mit einer Verbindung der Formel III, in der B eine Dialkoxyphosphonylmethylgruppe ist (Horner-Reaktion), werden die Komponenten mit Hilfe einer Base und vorzugsweise in Gegenwart eines inerten organischen Lösungsmittels, z. B. mit Hilfe von Natriumhydrid in Benzol, Toluol, Dimethylformamid, Tetrahydrofuran, Dioxan oder 1,2-Dimethoxyalkan, oder auch mit Hilfe eines Natriumalkoholats in einem Alkanol, z. B. Natriummethylat in Methanol, in einem zwischen 0° und dem Siedepunkt des Reaktionsgemisches liegenden Temperaturbereich kondensiert.

Gemäß Verfahrensvariante a) erhält man Verbindungen der Formel I, in denen Z einen der Reste Z' darstellt.

Gemäß der Verfahrensvariante b) wird eine Verbindung der Formel IV, in der X Halogen, vorzugsweise Brom, ist, mit einer starken Base, wie Butyllithium, behandelt und mit Schwefeldioxid umgesetzt. Die Reaktion kann in einem inerten organischen Lösungsmittel, z. B. einem Äther wie Diäthyläther, oder einem Kohlenwasserstoff, wie Hexan, oder Gemischen davon durchgeführt werden, zweckmäßig bei Temperaturen unterhalb Raumtemperatur, z. B. bei etwa 0°C. Verfahrensvariante b) liefert Salze von Sulfinsäuren der Formel I, d. h. Verbindungen der Formel I in denen Z einen Rest $-SO_2^-M^+$ darstellt.

Gemäß Verfahrensvariante c) wird eine Verbindung der Formel V mit einer Base behandelt oder mit einem komplexen Metallhydrid reduziert. Geeignete Basen sind z. B. Alkalimetallhydroxide wie Kaliumhydroxid. Die Reaktion wird zweckmäßig in einem inerten Lösungsmittel unter Verwendung von alkoholischer Alkalihydroxidlösung bei Temperaturen bis zur Rückflußtemperatur des Reaktionsgemisches durchgeführt und liefert Verbindungen der Formel I mit Z=Mercapto. Geeignete komplexe Metallhydride sind solche, die bekanntermaßen für die Reduktion eines Esters zum Alkohol in Betracht kommen, wie insbesondere Lithiumaluminiumhydrid. Die Reaktion kann unter den für solche Esterreduktionen bekannten Bedingungen durchgeführt werden, z. B. in einem Lösungsmittel wie Diäthyläther oder Tetrahydrofuran bei Temperaturen von etwa 0° bis Rückflußtemperatur des Reaktionsgemisches.

Als funktionelle Abwandlung des Restes Z in einer Verbindung der Formel I kommen in Betracht die Überführung eines Sulfonsäure- oder Sulfinsäuresalzes in die entsprechende freie Säure oder einen Ester; die Überführung einer Sulfonsäure oder Sulfinsäure in ein mono- oder di-nieder-Alkylamid; die Überführung eines Sulfinsäuresalzes in ein Sulfon; die Oxidation eines Sulfids zum Sulfoxid und die Oxidation eines Sulfoxids zum Sulfon; die Umwandlung einer Hydroxy-nieder-alkylgruppe in eine Halogen-nieder-alkylgruppe und die Umwandlung der letzteren Gruppe in eine nieder-Alkenylgruppe; und die Alkylierung eines Mercaptans zum Alkylsulfid oder eine Michael-Addition mit einem geeigneten Michael-Akzeptor, wie Acrylsäuremethylester oder Methylvinylketon.

Ein Sulfonat oder Sulfinat, d. h. eine Verbindung der Formel I, in der Z $-SO_3^-M^+$ oder $SO_2^-M^+$ darstellt, kann durch Behandlung mit Säure, z. B. Mineralsäure, wie Salzsäure, in die entsprechende Sulfon- bzw. Sulfinsäure, d. h. eine Verbindung der Formel I mit Z=$SO_3H$ bzw. $SO_2H$, übergeführt werden. Die Umwandlung einer Sulfon- oder Sulfinsäure in ein Amid, d. h. eine Verbindung der Formel I, in der Z mono- oder di-nieder-Alkylamino-sulfonyl oder -sulfinyl darstellt, kann in an sich bekannter Weise erfolgen, z. B. durch Überführung der Säure in ein Säurehalogenid (z. B. mittels Thionylchlorid) und Umsetzung des Säurehalogenids mit einem mono- oder di-nieder-Alkylamin. Ester, d. h., Verbindungen der Formel I, in denen Z nieder-Alkoxy-sulfonyl oder -sulfinyl darstellt, können aus Sulfonaten oder Sulfinaten, d. h. Verbindungen der Formel I, in denen Z $-SO_3^-M^+$ bzw. $SO_2^-M^+$ ist, durch Umsetzung mit einem Alkylierungsmittel, wie Triäthyloxonium-tetrafluoroborat oder Dialkylsulfat, z. B. Dimethyl- oder Diäthylsulfat, erhalten werden. Die Oxidation eines Sulfids, d. h. einer Verbindung der Formel I in der Z nieder-Alkylthio darstellt, zu dem entsprechenden Sulfoxid (Z = nieder-Alkylsulfinyl) sowie die Oxidation eines Sulfoxids zu dem entsprechenden Sulfon (Z = nieder-Alkylsulfonyl) kann durch Behandlung mit Oxidationsmitteln, wie Persäuren, z. B. m-Chlorperbenzoesäure, durchgeführt werden. Ein Sulfid kann auch mit Perjodaten, z. B. $NaJO_4$ zum Sulfoxid oxidiert werden.

Ein nieder-Alkyl-thioäther oder ein nieder-Alkylsulfoxid oder -sulfon (Z = $-S(O)_mR$ = nieder-Alkyl) kann in ein entsprechendes nieder-Carbalkoxy-nieder-alkyl-Derivat, d. h. eine Verbindung der Formel I mit Z = $-S(O)_mR$ und R = nieder-Carbalkoxy-nieder-alkyl umgewandelt werden, z. B. dadurch, daß man die nieder-Alkylverbindung, z. B. ein nieder-Alkyl-sulfon mit einer starken Base, wie Butyllithium, behandelt und die metallorganische Verbindung mit einem nieder-Carbalkoxy-nieder-alkyl-halogenid, z. B. Chloressigsäure-äthylester oder mit einem Alkanoylierungsmittel, wie einem Alkancarbonsäureester oder einem Alkanoylhalogenid umsetzt.

Ein Hydroxy-nieder-alkyl-thioäther, -sulfoxid oder -sulfon kann durch Austausch der Hydroxygruppe gegen ein Halogenatom, z. B. mittels Halogenierungsmitteln wie Thionylchlorid, in das entsprechende Halogen-nieder-alkylderivat (Z = $-S(O)_mR$, R = Halogenniederalkyl) übergeführt werden, aus dem durch Behandlung mit Basen, z. B. Aminen wie Triäthylamin, ein nieder-Alkenylthioäther bzw. ein nieder-Alkenylsulfoxid oder -sulfon erhalten werden kann (Z = $-S(O)_mR$, R = nieder-Alkenyl).

Die Ausgangsstoffe der Formeln II, III, IV und V können, soweit ihre Herstellung nicht bekannt oder nachstehend beschrieben ist, in Analogie zu bekannten oder den nachstehend beschriebenen Verfahren hergestellt werden.

Die Verfahrensprodukte der Formel I und ihre pharmazeutisch verträglichen Salze stellen pharmakodynamisch wertvolle Verbindungen dar. Sie können zur topischen und systemischen Therapie von benignen und malignen Neoplasien, von prämalignen Läsionen, sowie ferner auch zur systemischen und topischen Prophylaxe der genannten Affektion verwendet werden.

Sie sind des weiteren für die topische und systemische Therapie von Akne, Psoriasis und anderen mit einer verstärkten oder pathologisch veränderten Verhornung einhergehenden Dermatosen, wie auch von entzündlichen und allergischen dermatologischen Affektionen geeignet. Die Verfahrensprodukte der Formel I und ihre pharmazeutisch verträglichen Salze können ferner auch zur Bekämpfung von Schleimhauterkrankungen mit entzündlichen oder degenerativen bzw. metaplastischen Veränderungen eingesetzt werden.

Die neuen, zur Klasse der Retinoide gehörenden Verbindungen zeichnen sich gegenüber bekannten Retinoiden z. B. dadurch aus, daß sie bei den Versuchstieren einen geringeren Gewichtsverlust bewirken, der häufig bei der Verabreichung von Retinoiden als Nebenwirkung (A-Hypervitaminose) auftritt.

Die tumorhemmende Wirkung der Verfahrensprodukte ist signifikant. Man beobachtet im Papillomtest bei Mäusen [Europ. J. Cancer 10, 732 (1974)] eine Zurückbildung der mit Dimethylbenzanthracen und Krotonöl induzierten Tumoren. Beispielsweise nehmen die Durchmesser der Papillome im Verlauf von 2 Wochen bei intraperitonealer Applikation von Äthyl p-[2-(5,6,7,8-Tetrahydro-5,5,8,8-tetramethyl-2-naphthyl)-propenyl]phenylsulfon

bei 12,5 mg/kg/Woche um 56%
bei 6,25 mg/kg/Woche um 36%
bei 3 mg/kg/Woche um 12%

ab.

Zur Behandlung dieser Erkrankungen werden die erfindungsgemäßen Verbindungen oral, zweckmäßig in einer Dosierung bei Erwachsenen von etwa 5200 mg pro Tag, vorzugsweise 10—50 mg/Tag, verabreicht. Eine etwaige Überdosierung kann sich in Form einer Vit-A-Hypervitaminose äußern, und ist an deren Symptomen (Hautschuppung, Haarausfall) leicht zu erkennen.

Die Dosis kann als Einzeldosis oder auf mehrere Teildosen verteilt verabreicht werden.

Die Verbindungen der Formel I und pharmazeutisch verträgliche Salze können deshalb als Heilmittel, z. B. in Form pharmazeutischer Präparate, Anwendung finden.

Die zur systematischen Anwendung dienenden Präparate können z. B. dadurch hergestellt werden, daß man eine Verbindung der Formel I oder ein pharmazeutisch verträgliches Salz davon als wirksamen Bestandteil nichttoxischen, inerten, an sich in solchen Präparaten üblichen festen oder flüssigen Trägern zufügt.

Die Mittel können enteral, parenteral oder topisch verabreicht werden. Für die enterale Applikation eignen sich z. B. Mittel in Form von Tabletten, Kapseln, Dragées, Sirupen, Suspensionen, Lösungen und Suppositorien. Für die parenterale Applikation sind Mittel in Form von Infusions- oder Injektions-Lösungen geeignet.

Die Dosierungen, in denen die erfindungsgemäßen Produkte verabreicht werden, können je nach Anwendungsart und Anwendungsweg sowie nach den Bedürfnissen der Patienten variieren.

Die erfindungsgemäßen Produkte können in einer oder mehreren Dosierungen verabreicht werden. Eine bevorzugte Darreichungsform sind Kapseln mit einem Gehalt von ca. 5, 20 oder 50 mg Wirkstoff.

Die Präparate können inerte oder auch pharmakodynamisch aktive Zusätze enthalten. Tabletten oder Granulate z. B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z. B. aus Wasser, Gelatine, Milchzucker, Stärke, Magnesiumstearat, Talkum, Gummi arabicum, Polyalkylenglykolen und dgl. bestehen. Voraussetzung ist, daß alle bei der Herstellung der Präparate verwendeten Hilfsstoffe untoxisch sind.

Zur topischen Anwendung werden die erfindungsgemäßen Produkte zweckmäßig in Form von Salben, Tinkturen, Crèmen, Lösungen, Lotionen, Sprays, Suspensionen und dgl. verwendet. Bevorzugt sind Salben und Crèmen sowie Lösungen. Diese zur topischen Anwendung bestimmten Präparate können dadurch hergestellt werden, daß man die erfindungsgemäßen Produkte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmäßig ca. 0,05 bis ca. 5%ige, vorzugsweise 0,1 bis 1%ige Lösungen, Salben oder Crèmen geeignet.

Den Präparaten kann gegebenenfalls ein Antioxydationsmittel, z. B. Tocopherol, N-Methyl-$\gamma$-tocopheramin sowie butyliertes Hydroxyanisol oder butyliertes Hydroxytoluol beigemischt sein.

Die nachfolgenden Beispiele erläutern die Erfindung.

## Beispiel 1

4,05 g Natriumhydrid (50% Suspension in Mineralöl) werden mit absolutem Pentan gewaschen, im Wasserstrahlvakuum getrocknet und in 100 ml Dimethylformamid suspendiert. Bei 0°C tropft man eine Suspension von 43 g [1-(5,6,7,8-Tetraydro-5,5,8,8-tetramethyl-2-naphthyl)äthyl]-triphenylphosphoniumbromid hinzu und läßt 1 Stunde bei 0°C rühren. Bei der gleichen Temperatur tropft man zu der so erhaltenen tiefroten Lösung eine Suspension von 16 g des Natriumsalzes der p-Formyl-benzolsulfonsäure in 200 ml Dimethylformamid. Nach 3stündigem Rühren bei Raumtemperatur gießt man die

braunrote Reaktionslösung auf Eis und dampft im Wasserstrahlvakuum zur Trockene ein. Der kristalline, graue Rückstand wird in Essigsäureäthylester aufgeschlämmt, abgesaugt und mehrfach mit Essigester gewaschen. Zweimaliges Umkristallisieren des Rückstandes aus siedendem Wasser ergibt 10 g Natrium p-[2-(5,6,7,8-tetrahydro-5,5,8,8-tetramethyl-2-naphthyl)propenyl]benzolsulfonat mit einem Zersetzungspunkt, der über 300°C liegt.

Beispiel 2

2,0 g des nach Beispiel 1 erhaltenen Natriumsulfonates werden in 70 ml halbkonzentrierter Salzsäure suspendiert, kurz erwärmt und im Eisbad abgekühlt. Der Niederschlag wird abgesaugt, gewaschen mit halbkonzentrierter Salzsäure und Wasser und am Hochvakuum bei 50°C getrocknet. Man erhält 1,9 g p-[2-(5,6,7,8-Tetrahydro-5,5,8,8-tetramethyl-2-naphthyl)propenyl]benzolsulfonsäure, Schmelzpunkt 103—110°C (Zersetzung).

Beispiel 3

4,8 g des nach Beispiel 1 erhaltenen Natriumsulfonates werden in 50 ml Dimethylformamid suspendiert und mit 2,0 g Thionylchlorid versetzt. Nach halbstündigem Rühren bei Raumtemperatur gibt man zu der gelben Suspension 15 ml Äthylamin und rührt noch 1 Stunde bei Raumtemperatur. Das Reaktionsgemisch wird auf Eis gegossen, mit Essigester extrahiert, die organische Phase mit gesättigter Kochsalzlösung gewaschen, getrocknet über Natriumsulfat und eingedampft: Man erhält ein braunes Öl, das mit einem Gemisch aus Hexan und Äther (1 : 1) über eine kurze Säule mit Kieselgel filtriert und anschließend aus Hexan/Äther umkristallisiert wird. Man erhält 1,6 g N-Äthyl-p-[2-(5,6,7,8-tetrahydro-5,5,8,8-tetramethyl-2-naphthyl)propenyl]benzolsulfonamid (farblose Kristalle), Schmelzpunkt 137—138°C.

Beispiel 4

1,5 g des nach Beispiel 1 erhaltenen Natriumsulfonates werden in 120 ml Methylenchlorid suspendiert und mit einer Lösung von 760 mg Triäthyloxonium-tetrafluoroborat in 15 ml Methylenchlorid versetzt. Man rührt 45 Minuten bei Raumtemperatur, saugt den Niederschlag ab, wäscht mit Äther und dampft das Filtrat ein. Nach dem Umkristallisieren aus Isopropyläther erhält man 1,3 g Äthyl p-[2-(5,6,7,8-tetrahydro-5,5,8,8-tetramethyl-2-naphthyl)propenyl]benzolsulfonat in glänzenden Plättchen, Schmelzpunkt 155—157°C.

Beispiel 5

13,0 g 6-(p-Brom-$\alpha$-methylstyryl)-1,2,3,4-tetrahydro-1,1,4,4-tetramethylnaphthalin werden in 300 ml absolutem Äther gelöst. Unter Eiskühlung tropft man 20 ml einer 2 N-Lösung von Butyllithium in Hexan hinzu und rührt weitere 1,5 Stunden bei 0°C. Danach leitet man 30 Minuten einen kräftigen Schwefeldioxidstrom in das Reaktionsgemisch ein, gießt auf ein Gemisch aus Wasser und gesättigter Natriumcarbonatlösung, extrahiert die so erhaltene wäßrige Suspension 2mal mit Äther, filtriert die wäßrige Lösung und trocknet den farblosen Niederschlag bei 80°C im Hochvakuum. Nach dem Umkristallisieren aus siedendem Wasser erhält man 7,1 g Natrium-p-[2-(5,6,7,8-tetrahydro-5,5,8,8-tetramethyl-2-naphthyl)propenyl]benzolsulfinat, Schmelzpunkt 312—320°C (Zersetzung).

Das als Ausgangsprodukt verwendete 6-(p-Brom-$\alpha$-methylstyryl)-1,2,3,4-tetrahydro-1,1,4,4-tetramethylnaphthalin läßt sich wie folgt herstellen:

50 g [1-(5,6,7,8-Tetrahydro-5,5,8,8-tetramethyl-2-naphthyl)-äthyl]-triphenylphosphoniumbromid werden mit 13,9 g p-Brom-benzaldehyd in 400 ml Butylenoxid 3 Stunden am Rückfluß erhitzt. Man kühlt ab, gießt auf 500 ml eines Methanol/Wasser-Gemisches (6 : 4), extrahiert 3mal mit Hexan, wäscht die organische Phase 2mal mit Wasser, trocknet über Natriumsulfat und dampft ein. Das so erhaltene gelb-orange Öl wird aus Essigester umkristallisiert und ergibt 14,5 g 6-(p-Brom-$\alpha$-methylstyryl)-1,2,3,4-tetramethylnaphthalin in farblosen Kristallen, Schmelzpunkt 133—136°C.

Beispiel 6

8,5 g des nach Beispiel 5 erhaltenen Natriumsulfinates werden in 350 ml absolutem Methylenchlorid suspendiert und unter Eiskühlung mit einer Lösung von 4,6 g Triäthyloxonium-tetrafluoroborat in 20 ml Methylenchlorid versetzt. Man rührt 2 Stunden bei +5°C, saugt den Niederschlag ab, wäscht mit Methylenchlorid nach und dampft das Filtrat ein. Das farblose Öl wird über Kieselgel filtriert (Eluierungsmittel Hexan/Äther = 4 : 1) und ergibt nach dem Umkristallisieren aus Hexan/Äther 4,8 g Äthyl-p-[2-(5,6,7,8-tetrahydro-5,5,8,8-tetramethyl-2-naphthyl)propenyl]benzolsulfinat in farblosen Kristallen, Schmelzpunkt 99–101°C.

## Beispiel 7

2,5 g des nach Beispiel 5 erhaltenen Natriumsulfinates werden in 60 ml absolutem Dimethylformamid suspendiert und mit 1,0 g Äthyljodid versetzt. Man rührt noch ca. 6 Stunden, bis man eine klare, gelbliche Lösung erhält, verdünnt mit viel Wasser und extrahiert mit Essigester. Die organische Phase wird mehrmals mit Wasser gewaschen, über Natriumsulfat getrocknet und eingedampft. Nach dem Umkristallisieren aus Hexan/Essigester erhält man 1,9 g Äthyl-p-[2-(5,6,7,8-tetrahydro-5,5,8,8-tetramethyl-2-naphthyl)-propenyl]-phenylsulfon in farblosen Kristallen, Schmelzpunkt 158–161°C.

In Analogie zu der oben beschriebenen Arbeitsweise erhält man

aus Natrium-p-[2-(5,6,7,8-tetrahydro-5,5,8,8-tetramethyl-2-naphthyl)propenyl]benzolsulfinat und Methyljodid Methyl-p-[2-(5,6,7,8-tetrahydro-5,5,8,8-tetramethyl-2-naphthyl)-propenyl]-phenylsulfon, Schmelzpunkt 176–178°C;

aus Natrium-p-[2-(5,6,7,8-tetrahydro-5,5,8,8-tetramethyl-2-naphthyl)propenyl]benzolsulfinat und Allylbromid Allyl-p-[2-(5,6,7,8-tetrahydro-5,5,8,8-tetramethyl-2-naphthyl)propenyl]-phenylsulfon, Schmelzpunkt 140–141°C;

aus Natrium-p-[2-(5,6,7,8-tetrahydro-5,5,8,8-tetramethyl-2-naphthyl)propenyl]benzolsulfinat und 2,2,2-Trifluoräthyljodid 2,2,2-Trifluoräthyl-p-[2-(5,6,7,8-tetrahydro-5,5,8,8-tetramethyl-2-naphthyl)-propenyl]-phenylsulfon, Schmelzpunkt 158–159°C.

## Beispiel 8

18,1 g [1-(5,6,7,8-Tetrahydro-5,5,8,8-tetramethyl-2-naphthyl)-äthyl]-triphenylphosphoniumbromid werden mit 5,0 g p-Äthylsulfonyl-benzaldehyd in 250 ml Butylenoxid 3,5 Stunden am Rückfluß erhitzt. Man kühlt ab, gießt auf 500 ml Methanol/Wasser (6 : 4), extrahiert 3mal mit Hexan, wäscht die organische Phase mit Wasser, trocknet über Natriumsulfat und dampft ein. Das Rohprodukt wird über Kieselgel filtriert (Eluierungsmittel Hexan/Essigester = 1 : 1) und aus Hexan/Essigester umkristallisiert. Man erhält 9,2 g Äthyl-p-[2-(5,6,7,8-tetrahydro-5,5,8,8-tetramethyl-2-naphthyl)-propenyl]-phenylsulfon in farblosen Kristallen, Schmelzpunkt 158–161°C.

Der als Ausgangsprodukt verwendete p-Äthylsulfonylbenzaldehyd läßt sich wie folgt herstellen:

7,8 g Natriumhydrid (50% Suspension in Mineralöl) werden mit absolutem Pentan gewaschen, im Wasserstrahlvakuum getrocknet und in 100 ml Dimethylformamid suspendiert. Unter Eiskühlung tropft man 11,3 g Äthylmercaptan zu, läßt bei Raumtemperatur 1 Stunde rühren und tropft anschließend eine Lösung von 25,0 g 4-Brombenzaldehyd in 100 ml Dimethylformamid zu. Nach 10 Minuten gießt man auf Eis, extrahiert mit Essigester, wäscht die organische Phase mit 2 N-Salzsäure und Wasser, trocknet über Natriumsulfat und dampft ein. Nach Filtration des öligen Rückstandes über Kieselgel (Eluierungsmittel Hexan/Äther = 4 : 1) erhält man 17,5 g 4-Thioäthylbenzaldehyd als dünnflüssiges, schwach gelbes Öl.

10 g 4-Thioäthylbenzaldehyd werden in 150 ml Methylenchlorid gelöst und unter Eiskühlung nach und nach mit 22 g m-Chlorperbenzoesäure versetzt. Das Reaktionsgemisch wird mit Methylenchlorid verdünnt, 2mal mit eiskalter Kaliumcarbonat-Lösung und Wasser extrahiert, über Natriumsulfat getrocknet und eingedampft. Nach dem Umkristallisieren aus Hexan/Äther erhält man 9,1 g 4-Äthylsulfonylbenzaldehyd in farblosen Kristallen, Schmelzpunkt 107–109°C.

## Beispiel 9

In Analogie zu der im Beispiel 8 beschriebenen Arbeitsweise erhält man

aus 1-(1,1,3,3-Tetramethyl-5-indanyl)äthyl-triphenylphosphoniumbromid und 4-Äthylsulfonylbenzaldehyd Äthyl-p-[2-(1,1,3,3-tetramethyl-5-indanyl)propenyl]-phenylsulfon, Schmelzpunkt 125–127°C;

aus [1-(5,6,7,8-Tetrahydro-5,5,8,8-tetramethyl-2-naphthyl)äthyl]-triphenylphosphoniumbromid und 4-n-Propylsulfonyl-benzaldehyd Propyl-p-[2-(5,6,7,8-tetrahydro-5,5,8,8-tetramethyl-2-naphthyl)propenyl]-phenylsulfon, Schmelzpunkt 151–152°C;

aus [1-(5,6,7,8-Tetrahydro-5,5,8,8-tetramethyl-2-naphthyl)äthyl]-triphenylphosphoniumbromid und 4-Isopropylsulfonylbenzaldehyd Isopropyl-p-[2-(5,6,7,8-tetrahydro-5,5,8,8-tetramethyl-2-naphthyl)propenyl]-phenylsulfon, Schmelzpunkt 146–147°C;

aus [1-(5,6,7,8-Tetrahydro-5,5,8,8-tetramethyl-2-naphthyl)äthyl]-triphenylphosphoniumbromid

und 4-Isobutylsulfonylbenzaldehyd 1-Methylpropyl-p-[2-(5,6,7,8-tetrahydro-5,5,8,8-tetramethyl-2-naphthyl)propenyl]-phenylsulfon, Schmelzpunkt 143—144°C;

aus [1-(5,6,7,8-Tetrahydro-5,5,8,8-tetramethyl-2-naphthyl)äthyl]-triphenylphosphoniumbromid und 4-(2-Hydroxyäthylsulfonyl)-benzaldehyd 2-[[p-[2-(5,6,7,8-Tetrahydro-5,5,8,8-tetramethyl-2-naphthyl)propenyl]phenyl]sulfonyl]äthanol, Schmelzpunkt 131—133°C;

aus [1-(5,6,7,8-Tetrahydro-5,5,8,8-tetramethyl-2-naphthyl)äthyl]-triphenylphosphoniumbromid und 4-Äthylthio-benzaldehyd Äthyl-p-[2-(5,6,7,8-tetrahydro-5,5,8,8-tetramethyl-2-naphthyl)-propenyl]phenylsulfid, Schmelzpunkt 88—89°C;

aus [1-(5,6,7,8-Tetrahydro-5,5,8,8-tetramethyl-2-naphthyl)äthyl]-triphenylphosphoniumbromid und 4-Methylthiobenzaldehyd Methyl-p-[2-(5,6,7,8-tetrahydro-5,5,8,8-tetramethyl-2-naphthyl)propenyl]phenylsulfid, Schmelzpunkt 119—120°C.

Die Benzaldehydderivate können wie in Beispiel 8 angegeben durch Umsetzung von p-Brombenzaldehyd mit dem entsprechenden Mercaptan und anschließende Oxidation mit m-Chlorperbenzoesäure hergestellt werden.

Beispiel 10

6,4 g Äthyl-p-[2-(5,6,7,8-tetrahydro-5,5,8,8-tetramethyl-2-naphthyl)propenyl]phenylsulfid werden in 150 ml Methylenchlorid gelöst und bei 0°C innerhalb von 6 Stunden mit 3 g m-Chlorperbenzoesäure versetzt. Das Reaktionsgemisch wird noch 2 Stunden bei 0°C gerührt, mit Methylenchlorid verdünnt, 2mal mit eiskalter Kaliumcarbonat-Lösung und Wasser extrahiert, über Natriumsulfat getrocknet und eingedampft. Nach Chromatographie an Kieselgel (Eluierungsmittel Hexan/Essigester = 4 : 1) und Kristallisation aus Hexan/Essigester erhält man 3,1 g Äthyl-p-[2-(5,6,7,8-tetrahydro-5,5,8,8-tetramethyl-2-naphthyl)propenyl]-phenylsulfoxid, Schmelzpunkt 148—150°C.

In Analogie erhält man

aus Methyl-p-[2-(5,6,7,8-tetrahydro-5,5,8,8-tetramethyl-2-naphthyl)propenyl]phenylsulfid und m-Chlorperbenzoesäure Methyl-p-[2-(5,6,7,8-tetrahydro-5,5,8,8-tetramethyl-2-naphthyl)propenyl]phenylsulfoxid, Schmelzpunkt 155—156°C; und

aus Methyl-p-[2-(5,6,7,8-tetrahydro-5,5,8,8-tetramethyl-2-naphthyl)propenyl]phenylsulfoxid und m-Chlorperbenzoesäure Methyl-p-[2-(5,6,7,8-tetrahydro-5,5,8,8-tetramethyl-2-naphthyl)propenyl]phenylsulfon, Schmelzpunkt 176—178°C.

Beispiel 11

6,0 g 2-[[p-[2-(5,6,7,8-Tetrahydro-5,5,8,8-tetramethyl-2-naphthyl)propenyl]phenyl]sulfonyl]-äthanol werden in 150 ml Tetrahydrofuran gelöst und nach Zugabe von 1 ml Pyridin unter Eiskühlung langsam mit 1,7 g Phosphortribromid versetzt. Nach 15 Minuten gießt man auf Eis/Wasser, extrahiert mit Essigester, wäscht die organische Phase mit Wasser, trocknet über Natriumsulfat und dampft ein. Das so erhaltene bräunliche Öl wird in 200 ml Tetrahydrofuran gelöst und nach Zugabe von 70 ml Triäthylamin 6 Stunden auf 60°C erwärmt. Das Reaktionsgemisch wird auf Eis/Wasser gegossen, mit Essigester extrahiert, die organische Phase mit Wasser gewaschen, getrocknet und eingedampft. Nach Filtration über Kieselgel (Eluierungsmittel Hexan/Essigester = 4 : 1) und Kristallisation aus Hexan/Essigester erhält man 2,5 g Vinyl-p-[2-(5,6,7,8-tetrahydro-5,5,8,8-tetramethyl-2-naphthyl)propenyl]phenylsulfon, Schmelzpunkt 130—132°C.

Beispiel 12

4,5 g 2-[[p-[2-(5,6,7,8-Tetrahydro-5,5,8,8-tetramethyl-2-naphthyl)propenyl]phenyl]sulfonyl]-äthanol werden in 50 ml Acetonitril gelöst und mit 4 g Triphenylphosphin und 10 ml Tetrachlorkohlenstoff versetzt. Nach 4stündigem Kochen am Rückfluß gibt man erneut 2 g Triphenylphosphin und 2 ml Tetrachlorkohlenstoff zu und erhitzt weitere 3 Stunden am Rückfluß. Man kühlt ab, gießt auf Wasser, extrahiert mit Essigester, trocknet und dampft ein. Nach Chromatographie am Kieselgel (Eluierungsmittel Hexan/Essigester = 4 : 1) und Kristallisation aus Pentan/Äther erhält man 2,2 g 2-Chloräthyl-p-[2-(5,6,7,8-tetrahydro-5,5,8,8-tetramethyl-2-naphthyl)propenyl]phenylsulfon, Schmelzpunkt 136—138°C.

Beispiel 13

5,3 g Methyl-p-[2-(5,6,7,8-tetrahydro-5,5,8,8-tetramethyl-2-naphthyl)propenyl]phenylsulfon werden in 100 ml absolutem Tetrahydrofuran gelöst und bei —75°C mit 9 ml einer 2molaren Lösung von

Butyl-Lithium in Hexan versetzt. Nach 30minütigem Rühren bei —75°C gibt man 1,8 g Chlorameisensäureäthylester zu und rührt weitere 2 Stunden bei Raumtemperatur. Das Reaktionsgemisch wird auf Eiswasser gegossen, mit Essigester extrahiert, getrocknet und eingedampft. Nach Filtration über Kieselgel (Eluierungsmittel Hexan/Essigester = 4 : 1) und Kristallisation aus Hexan/Essigester erhält man 2,5 g Äthyl[[p-[2-(5,6,7,8-tetrahydro-5,5,8,8-tetramethyl-2-naphthyl)propenyl]phenyl]sulfonyl]-acetat, Schmelzpunkt 117—118°C.

Beispiel 14

99 g [1-(5,6,7,8-Tetrahydro-5,5,8,8-tetramethyl-2-naphthyl)-äthyl]-triphenylphosphoniumbromid werden mit 25,2 g S-(p-Formylphenyl)dimethylthiocarbamat in 1 l Butylenoxid 40 Stunden am Rückfluß erhitzt. Man kühlt ab, gießt auf 1 l Methanol/Wasser (6 : 4), extrahiert 3mal mit Hexan, wäscht die organische Phase mit Wasser, trocknet über Natriumsulfat und dampft ein. Das Rohprodukt wird über Kieselgel filtriert (Eluierungsmittel Hexan/Essigester 4 : 1) und aus Essigester/Hexan umkristallisiert. Man erhält 39 g S-[p-[2-(5,6,7,8-Tetrahydro-5,5,8,8-tetramethyl-2-naphthyl)propenyl]phenyl]dimethylthiocarbamat in farblosen Kristallen, Schmelzpunkt 107—109°C.

Das als Ausgangsmaterial verwendete S-(p-Formylphenyl)-dimethylthiocarbamat (Schmelzpunkt 78—80°C) kann aus p-Hydroxybenzaldehyd über O-(p-Formylphenyl)dimethylthiocarbamat (Schmelzpunkt 94—96°C) nach dem von M. S. Newmann und H. A. Karnes in J. Org. Chem. 31, 3980 (1966) angegebenen Verfahren hergestellt werden.

Beispiel 15

500 mg Lithiumaluminiumhydrid werden in 10 ml Tetrahydrofuran suspendiert und unter Eiskühlung tropfenweise mit einer Lösung von 5 g S-[p-[2-(5,6,7,8-Tetrahydro-5,5,8,8-tetramethyl-2-naphthyl)-propenyl]phenyl]dimethylthiocarbamat in 10 ml Tetrahydrofuran versetzt. Nach 2stündigem Rühren bei Raumtemperatur wird unter Eiskühlung durch Zutropfen von Wasser das überschüssige Lithiumaluminiumhydrid zerstört und nach Ansäuern der Lösung mit 1 N-Salzsäure mit Äther extrahiert. Die Ätherphase wird mit Wasser neutral gewaschen, über Natriumsulfat getrocknet und eingedampft. Man erhält 3,5 g p-[2-(5,6,7,8-Tetrahydro-5,5,8,8-tetramethyl-2-naphthyl)propenyl]thiophenol als farbloses, sehr oxidationsempfindliches Öl, das sich aus Hexan/Äther kristallisieren läßt, Schmelzpunkt 97—98°C.

Beispiel 16

500 mg Natriumhydrid (50% Suspension in Mineralöl) werden mit absolutem Pentan gewaschen, im Wasserstrahlvakuum getrocknet und in 10 ml Dimethylformamid suspendiert. Unter Eiskühlung tropft man eine Lösung von 3,3 g p-[2-(5,6,7,8-Tetrahydro-5,5,8,8-tetramethyl-2-naphthyl)-propenyl]thiophenol in 5 ml Dimethylformamid hinzu. Nach 1stündigem Rühren bei 0°C tropft man 3 g Äthyljodid hinzu, läßt auf Raumtemperatur kommen und rührt noch 3 Stunden nach. Die Reaktionslösung wird auf Eis gegossen, mit Äther extrahiert, die organische Phase mit 2 N-Salzsäure und Wasser gewaschen, getrocknet über Natriumsulfat und eingedampft. Das Rohprodukt wird über Kieselgel filtriert (Eluierungsmittel Hexan/Essigester = 9 : 1) und aus Hexan umkristallisiert. Man erhält 2,7 g Äthyl p-[2-(5,6,7,8-tetrahydro-5,5,8,8-tetramethyl-2-naphthyl)propenyl]phenylsulfid, Schmelzpunkt 88—89°C.

Beispiel 17

3,5 g p-[2-(5,6,7,8-Tetrahydro-5,5,8,8-tetramethyl-2-naphthyl)propenyl]thiophenol werden in 20 ml Dimethylformamid gelöst und mit 4 g Acrylsäuremethylester versetzt. Nach dem Zugeben von einigen Tropfen Triäthylamin rührt man 2 Stunden bei 60°C. Nach dem Abkühlen gießt man auf Eiswasser, extrahiert mit Äther, wäscht mit Wasser, trocknet und dampft ein. Das Rohprodukt wird über Kieselgel filtriert (Eluierungsmittel Hexan/2% Essigester) und aus Hexan/Äther kristallisiert. Man erhält 3,1 g Methyl-3-[[p-[2-(5,6,7,8-tetrahydro-5,5,8,8-tetramethyl-2-naphthyl)propenyl]phenyl]thio]propionat, Schmelzpunkt 90—92°C.

Durch Oxidation mit m-Chlorperbenzoesäure erhält man daraus Methyl-3-[[p-[2-(5,6,7,8-tetrahydro-5,5,8,8-tetramethyl-2-naphthyl)propenyl]phenyl]sulfonyl]propionat, Schmelzpunkt 118—120°C.

Beispiel 18

3,9 g Äthyl-p-[2-(5,6,7,8-tetrahydro-5,5,8,8-tetramethyl-2-naphthyl)propenyl]phenylsulfon werden

in 20 ml Tetrahydrofuran gelöst und bei −78°C mit 5,5 ml einer 2molaren Lösung von Butyllithium in Hexan versetzt. Nach 15 Minuten gibt man 370 mg Essigsäuremethylester hinzu. Nach 30minütigem Rühren bei −78°C gibt man erneut 2,75 ml der Butyllithium-Lösung hinzu und versetzt nach 15 Minuten mit 185 mg Essigsäuremethylester. Nach weiteren 30 Minuten wiederholt man den gleichen Vorgang durch Zugabe von 1,4 ml Butyllithium-Lösung und 93 mg Essigsäuremethylester. Man läßt auf Raumtemperatur kommen, gießt auf Eiswasser und extrahiert mit Äther. Nach dem Trocknen und Eindampfen der organischen Phase kristallisiert man aus Essigester/Hexan und erhält 2,6 g 3-[[p-[2-(5,6,7,8-Tetrahydro-5,5,8,8-tetramethyl-2-naphthyl)propenyl]phenyl]sulfonyl]-2-butanon, Schmelzpunkt 139—141°C.

In Analogie zu der oben beschriebenen Arbeitsweise erhält man aus Methyl-p-[2-(5,6,7,8-tetrahydro-5,5,8,8-tetramethyl-2-naphthyl)propenyl]phenylsulfon und Essigsäuremethylester 1-[[p-[2-(5,6,7,8-Tetrahydro-5,5,8,8-tetramethyl-2-naphthyl)propenyl]phenyl]sulfonyl]-1-propanon, Schmelzpunkt 140—141°C.

## Beispiel 19

5 g Natrium-p-[2-(5,6,7,8-tetrahydro-5,5,8,8-tetramethyl-2-naphthyl)propenyl]benzolsulfinat werden in 150 ml Benzol suspendiert und mit 1,3 g Chlormethylmethyläther versetzt. Nach 8stündigem Erwärmen auf 85°C dampft man das Lösungsmittel ab und chromatographiert den öligen Rückstand an Kieselgel (Eluierungsmittel Hexan/Essigester = 9 : 1). Nach dem Umkristallisieren aus Hexan/Essigester erhält man 1,1 g Methyl [[p-[2-(5,6,7,8-tetrahydro-5,5,8,8-tetramethyl-2-naphthyl)propenyl]-phenyl]sulfonyl]methyläther, Schmelzpunkt 154—156°C.

## Beispiel 20

4 g Natrium-p-[2-(5,6,7,8-tetrahydro-5,5,8,8-tetramethyl-2-naphthyl)propenyl]benzolsulfinat werden in einem Gemisch aus 80 ml Äthanol und 750 mg Essigsäure suspendiert. Man versetzt mit 750 mg Methylvinylketon und rührt 20 Stunden bei Raumtemperatur. Danach verdünnt man mit Wasser, extrahiert mit Äther, trocknet und dampft ein. Nach dem Umkristallisieren aus Hexan/Essigester erhält man 3,5 g 4-[[p-[2-(5,6,7,8-Tetrahydro-5,5,8,8-tetramethyl-2-naphthyl)propenyl]phenyl]sulfonyl]-2-butanon, Schmelzpunkt 135—136°C.

## Beispiel A

Eine Salbe mit 1% Wirkstoff kann aus folgenden Bestandteilen hergestellt werden:

| | |
|---|---|
| Wirkstoff, z. B. Äthyl-p-[2-(5,6,7,8-tetrahydro-5,5,8,8-tetramethyl-2-naphthyl)-propenyl]-phenylsulfon | 1,0 g |
| Vaseline, weiß | 35,0 g |
| Wachs, weiß | 4,0 g |
| Paraffinöl, dickflüssig | 18,0 g |
| DEHYMULS E*) | 7,0 g |
| Benzolsäure | 0,2 g |
| Wasser, entsalzt, ad | 100,0 g |

*) Hochmolekularer aliphatischer Ester, Lieferant: Deutsche Hydrierwerke.

## Beispiel B

Eine Salbe mit 0,1% Wirkstoff kann aus folgenden Bestandteilen hergestellt werden:

| | |
|---|---|
| Wirkstoff, z. B. Äthyl-p-[2-(5,6,7,8-tetrahydro-5,5,8,8-tetramethyl-2-naphthyl)-propenyl]-phenylsulfon | 1,0 g |
| Vaseline, weiß | 35,0 g |
| Wachs, weiß | 10,0 g |
| Paraffinöl, dickflüssig | 18,0 g |
| DEHYMULS E | 7,0 g |
| Benzolsäure | 0,2 g |
| Wasser, entsalzt, ad | 100,0 g |

Beispiel C

Eine Suspensionssalbe kann aus folgenden Bestandteilen hergestellt werden:

| | |
|---|---|
| Wirkstoff, z. B. Äthyl-p-[2-(5,6,7,8-tetrahydro-5,5,8,8-tetramethyl-2-naphthyl)-propenyl]-phenylsulfon | 0,3 g |
| Paraffinöl, dickflüssig | 36,7 g |
| Vaseline, weiß | 45,0 g |
| LUNACERA M*) | 15,0 g |
| Ricinusöl, gehärtet | 3,0 g |

*) Kohlenwasserstoffwachs; Lieferant: Lüneburger Wachsbleiche.

**Patentansprüche für die Vertragsstaaten: BE, CH, DE, FR, GB, IT, LI, LU, NL, SE**

1. Verbindungen der Formel

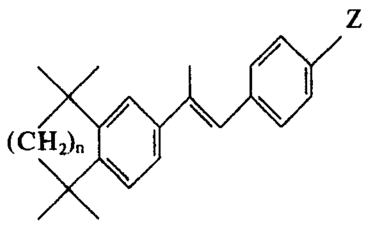

(I)

worin n 1 oder 2; Z Mercapto oder einen Rest $-S(O)_mR$, m 0, 1 oder 2; R nieder-Alkyl, nieder-Alkenyl, nieder-Alkoxy-nieder-alkyl, nieder-Alkanoyl-nieder-alkyl, Hydroxy-nieder-alkyl, Halogen-nieder-alkyl, nieder-Carbalkoxy-nieder-alkyl oder, falls m 1 oder 2 ist, auch nieder-Alkoxy, Hydroxy, mono- oder di-nieder-Alkylamino bedeutet,
und pharmazeutisch verträgliche Salze von Sulfon- und Sulfinsäuren der Formel I.

2. Verbindungen gemäß Anspruch 1, in denen R nieder-Alkyl, nieder-Alkenyl, Hydroxy-nieder-alkyl, Halogen-nieder-alkyl, nieder-Carbalkoxy-nieder-alkyl oder, falls m 1 oder 2 ist, auch nieder-Alkoxy, Hydroxy, mono- oder di-nieder-Alkylamino bedeutet.

3. Verbindungen gemäß Anspruch 1 oder 2, in denen n = 2 ist.

4. Verbindungen gemäß den Ansprüchen 1—3, in denen Z ein Rest $-SO_2R$ ist.

5. Verbindungen gemäß den Ansprüchen 1—4, in denen Z ein Rest $-S(O)_mR$ und R nieder-Alkyl, Hydroxy, nieder-Alkoxy, nieder-Alkylamino, Hydroxy-nieder-alkyl oder nieder-Carbalkoxy-nieder-alkyl ist.

6. Äthyl-p-[2-(5,6,7,8-tetrahydro-5,5,8,8-tetramethyl-2-naphthyl)propenyl]benzolsulfonat.

7. Äthyl-p-[2-(5,6,7,8-tetrahydro-5,5,8,8-tetramethyl-2-naphthyl)-propenyl]-phenylsulfon.

8. Äthyl-p-[2-(5,6,7,8-tetrahydro-5,5,8,8-tetramethyl-2-naphthyl)-propenyl]-phenylsulfoxid.

9. Äthyl-p-[2-(5,6,7,8-tetrahydro-5,5,8,8-tetramethyl-2-naphthyl)-propenyl]-phenylsulfid.

10. Isopropyl-p-[2-(5,6,7,8-tetrahydro-5,5,8,8-tetramethyl-2-naphthyl)-propenyl]-phenylsulfon.

11. Verbindungen der Formel I und deren pharmazeutisch verträglichen Salze als Heilmittel.

12. Verfahren zur Herstellung von Verbindungen der Formel I, dadurch gekennzeichnet, daß man

a) eine Verbindung der Formel

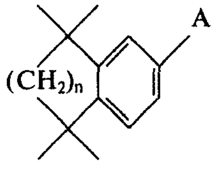

(II)

mit einer Verbindung der Formel

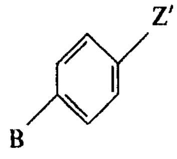

(III)

in denen n die oben angegebene Bedeutung hat, Z' $-SO_2^-M^+$, $-SO_3^-M^+$, nieder-Alkylthio, nieder-Alkylsulfinyl oder nieder-Alkylsulfonyl und entweder A eine Triarylphosphoniumäthylgruppe der

Formel

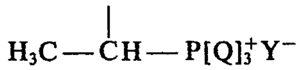

und B Formyl ist; oder A Acetyl und B eine Dialkoxyphosphonylmethylgruppe der Formel

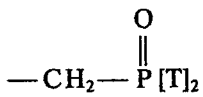

ist; wobei Q Aryl, T nieder-Alkoxy, $Y^-$ das Anion einer organischen oder anorganischen Säure und $M^+$ ein Kation bezeichnen,
umsetzt, oder

b) eine Verbindung der Formel

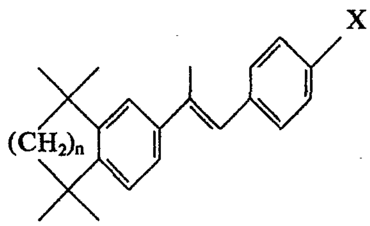

(IV)

in der n die obige Bedeutung hat und X Halogen ist,
in Gegenwart einer starken Base mit $SO_2$ umsetzt, oder

c) eine Verbindung der Formel

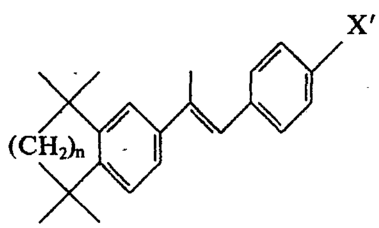

(V)

in der n die obige Bedeutung hat und X' di-nieder-Alkylcarbamoylthio ist,
mit einer Base behandelt oder mit einem komplexen Metallhydrid reduziert, und gewünschtenfalls in einer gemäß a, b oder c erhaltenen Verbindung der Formel I den Rest Z funktionell abwandelt.

13. Pharmazeutische Präparate auf der Basis einer Verbindung der Formel I oder eines ihrer pharmazeutisch verträglichen Salze.

**Patentansprüche für den Vertragsstaat: AT**

1. Verfahren zur Herstellung von

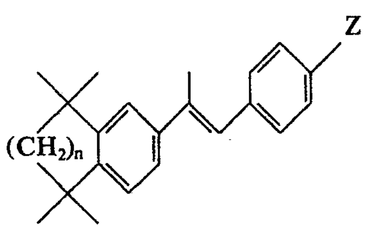

(I)

worin n 1 oder 2; Z Mercapto oder einen Rest $-S(O)_mR$, m 0, 1 oder 2; R nieder-Alkyl, nieder-Alkenyl, nieder-Alkoxy-nieder-alkyl, nieder-Alkanoyl-nieder-alkyl, Hydroxy-nieder-alkyl, Halogen-nieder-alkyl, nieder-Carbalkoxy-nieder-alkyl oder, falls m 1 oder 2 ist, auch nieder-Alkoxy, Hydroxy, mono- oder di-nieder-Alkylamino bedeutet,
und pharmazeutisch verträgliche Salze von Sulfon- und Sulfinsäuren der Formel I, dadurch gekennzeichnet, daß man

a) eine Verbindung der Formel

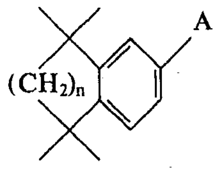

(II)

mit einer Verbindung der Formel

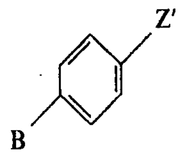

(III)

in denen n die oben angegebene Bedeutung hat, Z′ $-SO_2^-M^+$, $-SO_3^-M^+$, nieder-Alkylthio, nieder-Alkylsulfinyl oder nieder-Alkylsulfonyl und entweder A eine Triarylphosphoniumäthylgruppe der Formel

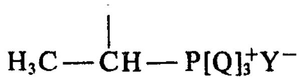

und B Formyl ist; oder A Acetyl und B eine Dialkoxyphosphonylmethylgruppe der Formel

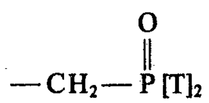

ist; wobei Q Aryl, T nieder-Alkoxy, $Y^-$ das Anion einer organischen oder anorganischen Säure und $M^+$ ein Kation bezeichnen,
umsetzt, oder

b) eine Verbindung der Formel

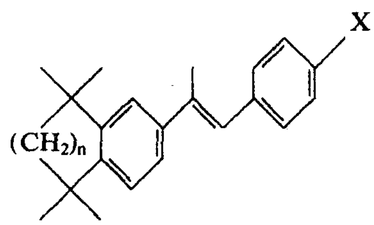

(IV)

in der n die obige Bedeutung hat und X Halogen ist,
in Gegenwart einer starken Base mit $SO_2$ umsetzt, oder

c) eine Verbindung der Formel

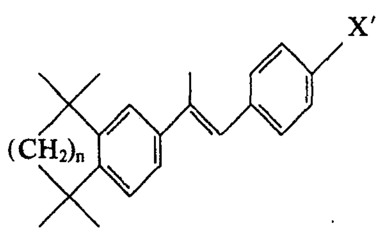

(V)

in der n die obige Bedeutung hat und X′ di-nieder-Alkylcarbamoylthio ist,
mit einer Base behandelt oder mit einem komplexen Metallhydrid reduziert, und gewünschtenfalls in einer gemäß a, b oder c erhaltenen Verbindung der Formel I den Rest Z funktionell abwandelt.

2. Verfahren nach Anspruch 1, dadurch gekennzeichnet, daß man Verbindungen der Formel I herstellt, in denen R nieder-Alkyl, nieder-Alkenyl, Hydroxy-nieder-alkyl, Halogen-nieder-alkyl, nieder-Carbalkoxy-nieder-alkyl oder, falls m 1 oder 2 ist, auch nieder-Alkoxy, Hydroxy, mono- oder di-nieder-Alkylamino bedeutet.

3. Verfahren nach Anspruch 1 oder 2, dadurch gekennzeichnet, daß man Verbindungen der Formel I herstellt, in denen n = 2 ist.

4. Verfahren nach den Ansprüchen 1–3, dadurch gekennzeichnet, daß man Verbindungen der Formel I herstellt, in denen Z ein Rest $-SO_2R$ ist.

5. Verfahren nach den Ansprüchen 1—4, dadurch gekennzeichnet, daß man Verbindungen der Formel I herstellt, in denen Z ein Rest $-S(O)_mR$ und R nieder-Alkyl, Hydroxy, nieder-Alkoxy, nieder-Alkylamino, Hydroxy-nieder-alkyl oder nieder-Carbalkoxy-nieder-alkyl ist.

6. Verfahren nach Anspruch 1, dadurch gekennzeichnet, daß man Äthyl-p-[2-(5,6,7,8-tetrahydro-5,5,8,8-tetramethyl-2-naphthyl)propenyl]benzolsulfonat herstellt.

7. Verfahren nach Anspruch 1, dadurch gekennzeichnet, daß man Äthyl-p-[2-(5,6,7,8-tetrahydro-5,5,8,8-tetramethyl-2-naphthyl)-propenyl]-phenylsulfon herstellt.

8. Verfahren nach Anspruch 1, dadurch gekennzeichnet, daß man Äthyl-p-[2-(5,6,7,8-tetrahydro-5,5,8,8-tetramethyl-2-naphthyl)-propenyl]-phenylsulfoxid herstellt.

9. Verfahren nach Anspruch 1, dadurch gekennzeichnet, daß man Äthyl-p-[2-(5,6,7,8-tetrahydro-5,5,8,8-tetramethyl-2-naphthyl)-propenyl]-phenylsulfid herstellt.

10. Verfahren nach Anspruch 1, dadurch gekennzeichnet, daß man Isopropyl-p-[2-(5,6,7,8-tetrahydro-5,5,8,8-tetramethyl-2-naphthyl)-propenyl]-phenylsulfon herstellt.

**Claims for the Contracting States: BE, CH, DE, FR, GB, IT, LI, LU, NL, SE**

1. Compounds of the formula

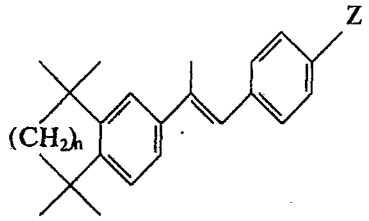

(I)

wherein n signifies 1 or 2; Z signifies mercapto or a residue $-S(O)_mR$, m signifies 0, 1 or 2; R signifies lower-alkyl, lower-alkenyl, lower-alkoxy-lower-alkyl, lower-alkanoyl-lower-alkyl, hydroxy-lower-alkyl, halogeno-lower-alkyl, lower-carbalkoxy-lower-alkyl or, when m is 1 or 2, also lower-alkoxy, hydroxy, mono- or di-lower-alkylamino,
and pharmaceutically compatible salts of sulphonic acids and sulphinic acids of formula I.

2. Compounds in accordance with claim 1, in which R signifies lower-alkyl, lower-alkenyl, hydroxy-lower-alkyl, halogeno-lower-alkyl, lower-carbalkoxy-lower-alkyl or, when m is 1 or 2, also lower-alkoxy, hydroxy, mono- or di-lower-alkylamino.

3. Compounds in accordance with claim 1 or 2, in which n = 2.

4. Compounds in accordance with claims 1—3, in which Z is a residue $-SO_2R$.

5. Compounds in accordance with claims 1—4, in which Z is a residue $-S(O)_mR$ and R is lower-alkyl, hydroxy, lower-alkoxy, lower-alkylamino, hydroxy-lower-alkyl or lower-carbalkoxy-lower-alkyl.

6. Ethyl p-[2-(5,6,7,8-tetrahydro-5,5,8,8-tetramethyl-2-naphthyl)propenyl]benzenesulphonate.

7. Ethyl p-[2-(5,6,7,8-tetrahydro-5,5,8,8-tetramethyl-2-naphthyl)-propenyl]-phenylsulphone.

8. Ethyl p-[2-(5,6,7,8-tetrahydro-5,5,8,8-tetramethyl-2-naphthyl)-propenyl]-phenylsulphoxide.

9. Ethyl-p-[2-(5,6,7,8-tetrahydro-5,5,8,8-tetramethyl-2-naphthyl)-propenyl]-phenylsulphide.

10. Isopropyl p-[2-(5,6,7,8-tetrahydro-5,5,8,8-tetramethyl-2-naphthyl)-propenyl]-phenylsulphone.

11. Compounds of formula I and their pharmaceutically compatible salts as medicaments.

12. A process for the manufacture of compounds of formula I, characterized by

a) reacting a compound of the formula

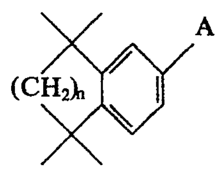

(II)

with a compound of the formula

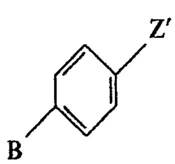

(III)

in which n has the significance given above, Z' is $-SO_2^-M^+$, $-SO_3^-M^+$, lower-alkylthio, lower-alkylsulphinyl or lower-alkylsulphonyl and either A is a triarylphosphoniumethyl group of the formula

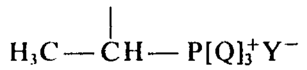

and B is formyl; or A is acetyl an B is a dialkoxyphosphonylmethyl group of the formula

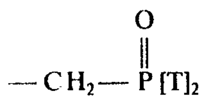

whereby Q denotes aryl, T denotes lower-alkoxy, $Y^-$ denotes the anion of an organic or inorganic acid and $M^+$ denotes a cation, or

b) reacting a compound of the formula

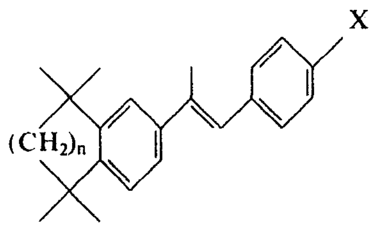

(IV)

in which n has the above significance and X is halogen, with $SO_2$ in the presence of a strong base, or

c) treating a compound of the formula

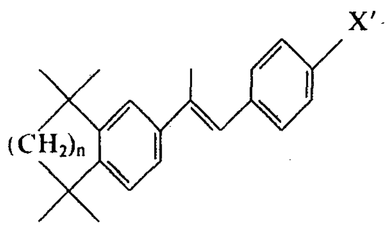

(V)

in which n has the above significance and X' is di-lower-alkylcarbamoylthio, with a base or reducing a compound of formula V with a complex metal hydride, and, if desired, functionally modifying the residue Z in a compound of formula I obtained in accordance with a, b or c.

13. Pharmaceutical preparations based on a compound of formula I or one of its pharmaceutically compatible salts.

**Claims for the Contracting State: AT**

1. A process for the manufacture of

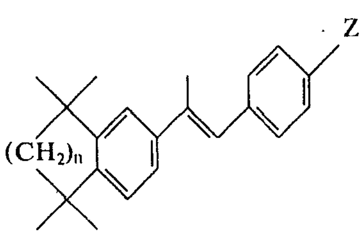

(I)

wherein n signifies 1 or 2; Z signifies mercapto or a residue $-S(O)_mR$, m signifies 0, 1 or 2; R signifies lower-alkyl, lower-alkenyl, lower-alkoxy-lower-alkyl, lower-alkanoyl-lower-alkyl, hydroxy-lower-alkyl, halogeno-lower-alkyl, lower-carbalkoxy-lower-alkyl or, when m is 1 or 2, also lower-alkoxy, hydroxy, mono- or di-lower-alkylamino,
and pharmaceutically compatible salts of sulphonic acids and sulphinic acids of formula I, characterized by

a) reacting a compound of the formula

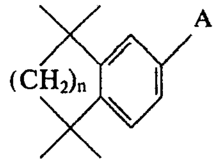

(II)

with a compound of the formula

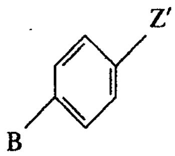

(III)

in which n has the significance given above, Z′ is $-SO_2^-M^+$, $-SO_3^-M^+$, lower-alkylthio, lower-alkylsulphinyl or lower-alkylsulphonyl and either A is a triarylphosphoniummethyl group of the formula

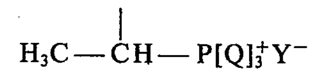

and B is formyl; or A is acetyl an B is a dialkoxyphosphonylmethyl group of the formula

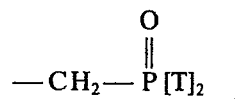

whereby Q denotes aryl, T denotes lower-alkoxy, Y denotes the anion of an organic or inorganic acid and $M^-$ denotes a cation, or

b) reacting a compound of the formula

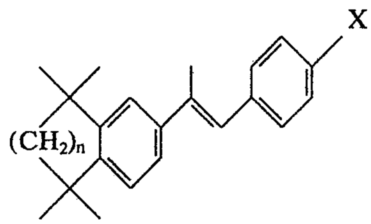

(IV)

in which n has the above significance and X is halogen, with $SO_2$ in the presence of a strong base, or

c) treating a compound of the formula

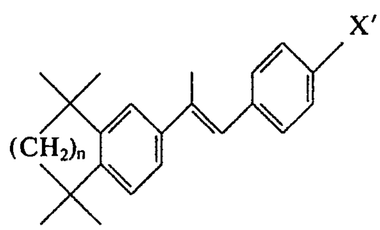

(V)

in which n has the above significance and X′ is di-lower-alkylcarbamoylthio, with a base or reducing a compound of formula V with a complex metal hydride, and, if desired, functionally modifying the residue Z in a compound of formula I obtained in accordance with a, b or c.

2. A process according to claim 1, characterized in that compounds of formula I in which R signifies lower-alkyl, lower-alkenyl, hydroxy-lower-alkyl, halogeno-lower-alkyl, lower-carbalkoxy-lower-alkyl or, when m is 1 or 2, also lower-alkoxy, hydroxy, mono- or di-lower-alkylamino are manufactured.

3. A process according to claim 1 or 2, characterized in that compounds of formula I in which n = 2 are manufactured.

4. A process according to claims 1—3, characterized in that compounds of formula I in which Z is a residue $-SO_2R$ are manufactured.

5. A process according to claims 1—4, characterized in that compounds of formula I in which Z is a

residue $-S(O)_mR$ and R is lower-alkyl, hydroxy, lower-alkoxy, lower-alkylamino, hydroxy-lower-alkyl or lower-carbalkoxy-lower-alkyl are manufactured.

6. A process according to claim 1, characterized in that ethyl p-[2-(5,6,7,8-tetrahydro-5,5,8,8-tetramethyl-2-naphthyl)propenyl]benzenesulphonate is manufactured.

7. A process according to claim 1, characterized in that ethyl p-[2-(5,6,7,8-tetrahydro-5,5,8,8-tetramethyl-2-naphthyl)-propenyl]-phenylsulphone is manufactured.

8. A process according to claim 1, characterized in that ethyl p-[2-(5,6,7,8-tetrahydro-5,5,8,8-tetramethyl-2-naphthyl)-propenyl]-phenylsulphoxide is manufactured.

9. A process according to claim 1, characterized in that ethyl p-[2-(5,6,7,8-tetrahydro-5,5,8,8-tetramethyl-2-naphthyl)-propenyl]-phenylsulphide is manufactured.

10. A process according to claim 1, characterized in that isopropyl p-[2-(5,6,7,8-tetrahydro-5,5,8,8-tetramethyl-2-naphthyl)-propenyl]-phenylsulphone is manufactured.

**Revendications pour les Etats contractants: BE, CH, DE, FR, GB, IT, LI, LU, NL, SE**

1. Composés de formule

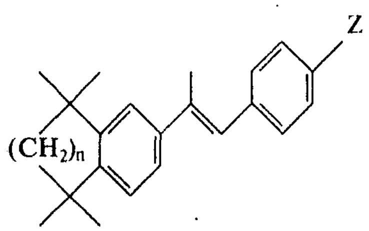

(I)

dans laquelle n est égal à 1 ou 2; Z représente un groupe mercapto ou un reste $-S(O)_mR$, m égal à 0, 1 ou 2; R représente un groupe alkyle inférieur, alcényle inférieur, (alcoxy inférieur)-alkyle inférieur, (alcanoyle inférieur)-alkyle inférieur, hydroxyalkyle inférieur, halogénoalkyle inférieur, (carbalcoxy inférieur)-alkyle inférieur ou bien encore, lorsque m est égal à 1 ou 2, alcoxy inférieur, hydroxy, mono- ou di-(alkyle inférieur)-amino,
et les sels acceptables pour l'usage pharmaceutique des acides sulfoniques et sulfiniques de formule I.

2. Composés selon la revendication 1, dans lesquels R représente un groupe alkyle inférieur, alcényle inférieur, hydroxyalkyle inférieur, halogénoalkyle inférieur, (carbalcoxy inférieur)-alkyle inférieur ou bien encore, lorsque m est égal à 1 ou 2, alcoxy inférieur, hydroxy, mono- ou di-(alkyle inférieur)-amino.

3. Composés selon la revendication 1 ou 2, dans lesquels $n = 2$.

4. Composés selon les revendications 1 à 3, dans lesquels Z représente un reste $-SO_2R$.

5. Composés selon les revendications 1 à 4, dans lesquels Z représente un reste $-S(O)_mR$ et R représente un groupe alkyle inférieur, hydroxy, alcoxy inférieur, alkylamino inférieur, hydroxyalkyle inférieur ou (carbalcoxy inférieur)-alkyle inférieur.

6. Le p-[2-(5,6,7,8-tétrahydro-5,5,8,8-tétraméthyl-2-naphtyl)-propényl]-benzène-sulfonate d'éthyle.

7. L'éthyl-p-[2-(5,6,7,8-tétrahydro-5,5,8,8-tétraméthyl-2-naphtyl)-propényl]-phénylsulfone.

8. L'éthyl-p-[2-(5,6,7,8-tétrahydro-5,5,8,8-tétraméthyl-2-naphtyl)-propényl]-phénylsulfoxyde.

9. Le sulfure d'éthyle et de p-[2-(5,6,7,8-tétrahydro-5,5,8,8-tétraméthyl-2-naphtyl)-propényl]-phényle.

10. L'isopropyl-p-[2-(5,6,7,8-tétrahydro-5,5,8,8-tétraméthyl-2-naphtyl)-propényl]-phénylsulfone.

11. Les composés de formule I et leurs sels acceptables pour l'usage pharmaceutique en tant que médicaments.

12. Procédé de préparation des composés de formule I caractérisé en ce que:

a) on fait réagir un composé de formule

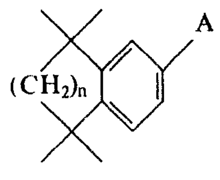

(II)

avec un composé de formule

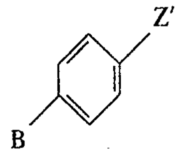

(III)

dans lesquelles n a les significations indiquées cidessus, Z' représente $-SO_2^-M^+$, $-SO_3^-M^+$, un groupe alkylthio inférieur, alkylsulfinyle inférieur ou alkyl-sulfonyle inférieur et ou bien A représente un groupe triarylphosphonium-éthyle de formule

$$H_3C-\overset{|}{C}H-P[Q]_3^+Y^-$$

et B représente un groupe formyle; ou bien A représente un groupe acétyle et B un groupe dialcoxyphosphonylméthyle de formule

$$-CH_2-\overset{\overset{O}{\|}}{P}[T]_2$$

Q représentant un groupe aryle, T un groupe alcoxy inférieur, $Y^-$ l'anion d'un acide minéral ou organique et $M^+$ un cation, ou bien

b) on fait réagir un composé de formule

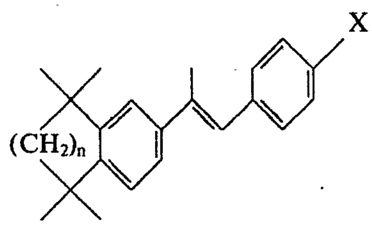

(IV)

dans laquelle n a les significations indiquées ci-dessus et X représente un halogène, en présence d'une base forte, avec $SO_2$, ou bien

c) on traite un composé de formule

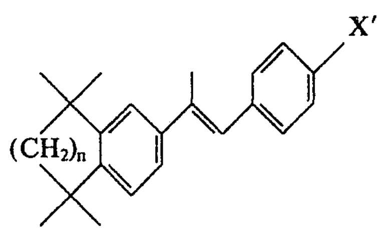

(V)

dans laquelle n a la signification ci-dessus et X' représente un groupe di-(alkyle inférieur)-carbamoylthio,

à l'aide d'une base ou on le réduit à l'aide d'un hydrure métallique complexe, et si on le désire, dans un composé de formule I obtenu ci-dessus sous a), b) ou c), on soumet le reste Z à une modification fonctionnelle.

13. Compositions pharmaceutiques à base d'un composé de formule I ou d'un sel d'un tel composé acceptable pour l'usage pharmaceutique.

**Revendications pour les Etat contractant: AT**

1. Procédé de préparation des composés de formule

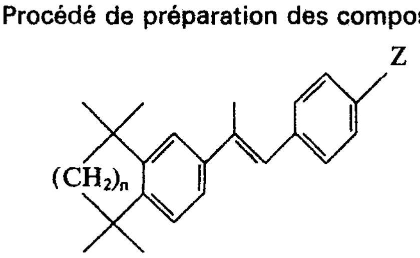

(I)

dans laquelle n est égal à 1 ou 2; Z représente un groupe mercapto ou un reste $-S(O)_mR$, m est égal à 0, 1 ou 2; R représente un groupe alkyle inférieur, alcényle inférieur, (alcoxy inférieur)-alkyle inférieur, (alcanoyle inférieur)-alkyle inférieur, hydroxyalkyle inférieur, halogénoalkyle inférieur, (carbalcoxy inférieur)-alkyle inférieur ou bien encore, lorsque m est égal à 1 ou 2, alcoxy inférieur, hydroxy, mono- ou di-(alkyle inférieur)-amino,
et des sels acceptables pour l'usage pharmaceutique des acides sulfoniques et sulfiniques de formule I, caractérisé en ce que:

a) on fait réagir un composé de formule

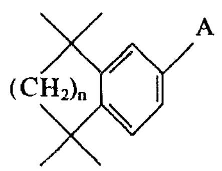

(II)

avec un composé de formule

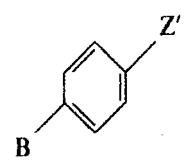

(III)

dans lesquelles n a les significations ci-dessus, Z' représente $-SO_2^-M^+$, $-SO_3^-M^+$, un groupe alkylthio inférieur, alkylsulfinyle inférieur ou alkyl-sulfonyle inférieur et/ou bien A représente un groupe triarylphosphonium-éthyle de formule

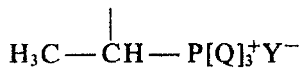

et B représente un groupe formyle; ou bien A représente un groupe acétyle et B un groupe dialcoxyphosphonylméthyle de formule

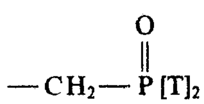

Q représentant un groupe aryle, T un groupe alcoxy inférieur, $Y^-$ l'anion d'un acide minéral ou organique et $M^+$ un cation, ou bien

b) on fait réagir un composé de formule

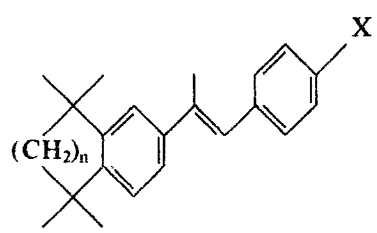

(IV)

dans laquelle n a les significations ci-dessus et X représente un halogène, en présence d'un base forte, avec $SO_2$, ou bien

c) on traite un composé de formule

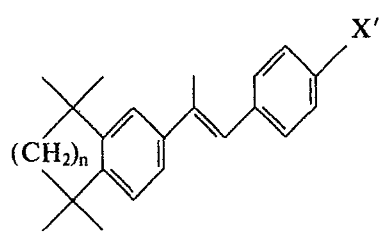

(V)

dans laquelle n a les significations ci-dessus et X' représente un groupe di-(alkyle inférieur)-carbamoylthio,
à l'aide l'dune base ou on le réduit à l'aide d'un hydrure métallique complexe, et si on le désire, dans

un composé de formule I obtenu ci-dessus sous a), b) ou c), on soumet le reste Z à une modification fonctionnelle.

2. Procédé selon la revendication 1, caractérisé en ce que l'on prépare des composés de formule I dans lesquels R représente un groupe alkyle inférieur, alcényle inférieur, hydroxyalkyle inférieur, halogénoalkyle inférieur, (carbalcoxy inférieur)-alkyle inférieur on bien encore, lorsque m est égal à 1 ou 2, alcoxy inférieur, hydroxy, mono- ou di-(alkyle inférieur)-amino.

3. Procédé selon la revendication 1 ou 2, caractérisé en ce que l'on prépare des composés de formule I dans lesquels n est égal à 2.

4. Procédé selon les revendications 1 à 3, caractérisé en ce que l'on prépare des composés de formule I dans lesquels Z représente un reste $-SO_2R$.

5. Procédé selon les revendications 1 à 4, caractérisé en ce que l'on prepare des composés de formule I dans lesquels Z représente un reste $-S(O)_mR$ et R représente un groupe alkyle inférieur, hydroxy, alcoxy inférieur, alkylamino inférieur, hydroxyalkyle inférieur ou (carbalcoxy inférieur)-alkyle inférieur.

6. Procédé selon la revendication 1, caractérisé en ce que l'on prépare le p-[2-(5,6,7,8-tétrahydro-5,5,8,8-tétraméthyl-2-naphtyl)-propényl]-benzène-sulfonate d'éthyle.

7. Procédé selon la revendication 1, caractérisé en ce que l'on prépare l'éthyl-p-[2-(5,6,7,8-tétrahydro-5,5,8,8-tétraméthyl-2-naphtyl)-propényl]-phénylsulfone.

8. Procédé selon la revendication 1, caractérisé en ce que l'on prépare l'éthyl-p-[2-(5,6,7,8-tétrahydro-5,5,8,8-tétraméthyl-2-naphtyl)-propényl]-phénylsulfoxyde.

9. Procédé selon la revendication 1, caractérisé en ce que l'on prépare le sulfure d'éthyle et de p-[2-(5,6,7,8-tétrahydro-5,5,8,8-tétraméthyl-2-naphtyl)-propényl]-phényle.

10. Procédé selon la revendication 1, caractérisé en ce que l'on prépare l'isopropyl-p-[2-(5,6,7,8-tétrahydro-5,5,8,8-tétraméthyl-2-naphtyl)-propényl]-phénylsulfone.